# EUROPÄISCHE PATENTSCHRIFT

(11) **EP 0 901 458 B1**
(45) Veröffentlichungstag und Bekanntmachung des Hinweises auf die Patenterteilung: **20.12.2000**
(21) Anmeldenummer: 97924942.2
(22) Anmeldetag: 12.05.1997
(51) Int. Cl.: C07C 213/02, C07C 215/80

(54) **VERFAHREN ZUR HERSTELLUNG VON 4,6-DIAMINO-RESORCIN-DIHYDROCHLORID**
PROCESS FOR PREPARING 4,6-DIAMINO-RESORCINOL-DIHYDROCHLORIDE
PROCEDE DE PREPARATION DE 4,6-DIAMINO-RESORCINE-DIHYDROCHLORURE

(30) Priorität: 22.05.1996 DE 19620589
(43) Veröffentlichungstag der Anmeldung: 17.03.1999
(73) Patentinhaber: BAYER AG, 51368 Leverkusen (DE)
(72) Erfinder: BEHRE, Horst, D-51519 Odenthal (DE); FIEGE, Helmut, D-51373 Leverkusen (DE); BLANK, Heinz, Ulrich, D-51519 Odenthal (DE); EYMANN, Wolfgang, D-51061 Köln (DE)
(86) Internationale Anmeldenummer: EP9702409
(87) Internationale Veröffentlichungsnummer: WO9744311

(56) Entgegenhaltungen:
- EP-A- 0 402 688
- EP-A- 0 710 644
- US-A- 5 414 130

## Beschreibung

Die vorliegende Erfindung betrifft ein Verfahren zur Herstellung von 4,6-Diamino-resorcin-dihydrochlorid aus 1,3-Dichlorbenzol durch Dinitrierung zum 1,3-Dichlor-4,6-dinitrobenzol, anschließende Umsetzung mit Natrium-benzylat zum 1,3-Dibenzyloxy-4,6-dinitrobenzol und katalytische Hydrierung.

4,6-Diamino-resorcin-dihydrochlorid ist ein wichtiger Monomerbaustein für Kunststoffe, insbesondere für Polybenzoxazole (Macromolecules 1981, 915).

In EP 0 402 688 ist die Synthese von 4,6-Diamino-resorcin aus 1,3-Dichlor-4,6-dinitrobenzol durch Umsetzung mit Natrium-benzylat zum 1,3-Dibenzyloxy-4,6-dinitrobenzol und anschließende katalytische Hydrierung beschrieben. Nachteilig an dem beschriebenen Verfahren ist die angegebene Ausführungsform der heterogenen katalytischen Hydrierung von 1,3-Dibenzyloxy-4,6-dinitrobenzol am Pd/A-Kohle-Kontakt als Ansatz-Hydrierung, die in der beschriebenen Form keine wirtschaftliche Rückführung des teuren Edelmetall-Kontaktes zuläßt. Ein weiterer Nachteil ist die unbefriedigende Produkt-Qualität, bedingt durch die beschriebene Ausführungsform der heterogenen Ansatz-Hydrierung und die geringe Stabilität von 4,6-Diamino-resorcin in freier Form, insbesondere in wasserhaltigen Lösungsmitteln. Ein weiterer Nachteil ist die geringe Löslichkeit von freiem 4,6-Diaminoresorcin in organischen Lösungsmitteln, was zu einer unwirtschaftlich hohen Verdünnung bei der Durchführung der katalytischen Hydrierung und der Produktisolierung führt.

Es wurde nun gefunden, daß man 4,6-Diamino-resorcin-dihydrochlorid aus 1,3-Dibenzyloxy-4,6-dinitrobenzol dadurch herstellen kann, daß man 1,3-Dibenzyloxy-4,6-dinitrobenzol an einem Edelmetall-Kontakt in einem zweiphasigen Gemisch aus verdünnter wäßriger Salzsäure und einem mit verdünnter wäßriger Salzsäure nicht oder nur begrenzt mischbaren organischen Lösungsmittel bei gegebenenfalls erhöhtem Druck und normaler oder erhöhter Temperatur durch eine katalytische Hydrierung, bevorzugt eine katalytische Pumphydrierung, zum 4,6-Diamino-resorcin-dihydrochlorid umsetzt.

Die Erfindung betrifft daher ein Verfahren zur Herstellung von 4,6-Diamino-resorcin-dihydrochlorid aus 1,3-Dibenzyloxy-4,6-dinitrobenzol, das dadurch gekennzeichnet ist, daß man 1,3-Dibenzyloxy-4,6-dinitrobenzol an einem Edelmetall-Kontakt mit Wasserstoff in einem zweiphasigen Gemisch aus verdünnter wäßriger Salzsäure und einem mit verdünnter wäßriger Salzsäure nicht mischbaren organischen Lösungsmittel bei normalem oder erhöhtem Druck und normaler oder erhöhter Temperatur katalytisch hydriert.

In das erfindungsgemäße Verfahren kann beispielsweise 1,3-Dibenzyloxy-4,6-dinitrobenzol eingesetzt werden, das gemäß EP 0 402 688 aus 1,3-Dichlor-4,6-dinitrobenzol durch Umsetzung mit Natrium-benzylat hergestellt wird.

In einer sehr wirtschaftlichen Ausführungsform läßt sich reines 1,3-Dibenzyloxy-4,6-dinitrobenzol aus 1,3-Dichlorbenzol gewinnen, wenn man
a) in einem ersten Schritt 1,3-Dichlorbenzol in wasserfreier Schwefelsäure, die 0 bis 10 Gew.-% freies SO₃ enthält, mit einer Salpetersäure, Schwefelsäure und 0,7 bis 1,5 Mol, bevorzugt 0,8 bis 1,2 Mol, besonders bevorzugt 0,9 bis 1,1 Mol SO₃ pro Mol Salpetersäure enthaltenden Mischsäure in einem Temperaturbereich von 0 bis 40°C umsetzt, wobei das Molverhältnis HNO₃ zu 1,3-Dichlorbenzol 2 bis 3 beträgt, und
b) in einem zweiten Schritt die erhaltenen 1,3-Dichlor-4,6/2,4-dinitrobenzol-Isomerengemische, die 0,1 bis 60 % 1,3-Dichlor-2,4-dinitrobenzol bezogen auf das Gesamtgewicht des Isomerengemisches enthalten, mit 2 bis 5 Mol Benzylalkohol und 2 bis 5 Äquivalenten einer starken Base in Gegenwart eines inerten Lösungsmittels umsetzt, wobei man hierzu stufenweise zunächst im Temperaturbereich von -15 bis +15°C und dann im Temperaturbereich von 20 bis 40°C arbeitet

Die Durchführung von Schritt a) kann in zwei verschiedenen Varianten erfolgen.

### Variante 1:

In eine Vorlage aus wasserfreier Schwefelsäure, die 0 bis 10 Gew-% freies SO₃ enthält, wird zunächst 1,3-Dichlorbenzol eingetropft und anschließend die Mischsäure, welche Salpetersäure, Schwefelsäure und mindestens 0,7 Mol SO₃ pro Mol Salpetersäure enthält, eindosiert. Die Dosierzeiten für 1,3-Dichlorbenzol betragen 5 Minuten bis 5 Stunden, die für die Mischsäure 15 Minuten bis 10 Stunden und hängen im wesentlichen von der Reaktionstemperatur und der Wirksamkeit des verwendeten Kühlaggregates sowie der Ansatzgröße ab. Bevorzugte Dosierzeiten für 1,3-Dichlorbenzol sind 15 bis 60 Minuten, für die Mischsäure 1 bis 3 Stunden.

### Variante 2:

In eine Vorlage aus wasserfreier Schwefelsäure, die 0 bis 10 Gew.-% freies SO₃ enthält, werden simultan 1,3-Dichlorbenzol und die Mischsäure, welche Salpetersäure, Schwefelsäure und mindestens 0,7 Mol SO₃ pro Mol Salpetersäure enthält, eindosiert. In dieser bevorzugten Variante beträgt die Simultan-Dosierzeit 15 Minuten bis 10 Stunden, bevorzugt 30 Minuten bis 3 Stunden, und hängt von der Reaktionstemperatur und der Wirksamkeit des verwendeten Kühlaggregates sowie der Ansatzgröße ab. Bei dieser bevorzugten Variante wird insbesondere bei der Vorlage von Schwefelsäure, die 0 bis 10 Gew.-% freies SO₃ enthält, die Bildung von 1,3-Dichlorbenzol-sulfonsäuren praktisch vollständig vermieden.

Die Temperatur von Schritt a) liegt im Bereich von 0 bis 40°C, bevorzugt 10 bis 30°C, insbesondere 15 bis 25°C.

Die erhaltenen Nitriergemische werden in an sich bekannter Form aufgearbeitet, beispielsweise durch Austragen auf Wasser oder ein Eis/Wasser-Gemisch, Filtration und Wasser-Wäsche.

Wesentlich für die Durchführung des Verfahrens in Schritt b) ist, daß man technische, in Schritt a) erhaltene 1,3-Dichlor-4,6/2,4-dinitrobenzol-Isomerengemische mit mindestens 2 Mol Benzylalkohol und mindestens 2 Äquivalenten einer starken Base, gegebenenfalls in Gegenwart eines inerten Lösungsmittels, umsetzt. Diese Isomerengemische enthalten im allgemeinen 0,1 bis 60 % 1,3-Dichlor-2,4-dinitrobenzol, bezogen auf das Gesamtgewicht des Gemisches (Rest: 4,6-Isomeres). Bevorzugt werden Gemische mit 5 bis 25 % 1,3-Dichlor-2,4-dinitrobenzol eingesetzt. Ganz besonders bevorzugt ist ein Gemisch aus 8 bis 15 % 1,3-Dichlor-2,4-dinitrobenzol und 85 bis 92 % 1,3-Dichlor-4,6-dinitrobenzol.

Das 1,3-Dichlor-4,6/2,4-dinitrobenzol-Isomerengemisch wird mit 2 bis 5 Mol, bevorzugt mit 2 bis 3 Mol Benzylalkohol und mit 2 bis 5 Äquivalenten einer starken Base umgesetzt. Als starke Basen werden eines oder mehrere Metalle aus der Gruppe der Alkalimetalle, wie Lithium, Natrium, Kalium, Rubidium oder Cäsium, bevorzugt Natrium oder Kalium, der Erdalkalimetalle, wie Magnesium, Calcium, Strontium oder Barium, bevorzugt Magnesium oder Calcium, der Alkalimetallhydroxide, wie Lithiumhydroxid, Natriumhydroxid, Kaliumhydroxid, Rubidiumhydroxid, Cäsiumhydroxid, bevorzugt Natriumhydroxid oder Kaliumhydroxid, der Erdalkalimetallhydroxide, wie Magnesiumhydroxid, Calciumhydroxid, Strontiumhydroxid oder Bariumhydroxid, bevorzugt Magnesiumhydroxid oder Calciumhydroxid, der Alkalimetallcarbonate, wie Lithiumcarbonat, Natriumcarbonat, Kaliumcarbonat, Rubidiumcarbonat oder Cäsiumcarbonat, bevorzugt Natriumcarbonat oder Kaliumcarbonat, und der Erdalkalimetallcarbonate, wie Magnesiumcarbonat, Calciumcarbonat, Strontiumcarbonat oder Bariumcarbonat, bevorzugt Magnesiumcarbonat oder Calciumcarbonat, eingesetzt. In bevorzugter Weise wird eine starke Base aus der Gruppe der Alkalimetalle, der Alkalimetallhydroxide und Alkalimetallcarbonate, in besonders bevorzugter Weise aus der Gruppe Natriummetall, Natriumhydroxid und Natriumcarbonat, eingesetzt. Ebenso können der Benzylalkohol und die Base auch in Form des vorgebildeten Alkoholates eingesetzt werden.

Die Temperatur-gestufte Reaktion von Schritt b) wird in der ersten Stufe im Temperaturbereich von -15 bis +15°C, bevorzugt 0 bis 15°C, insbesondere 5 bis 15°C, und in der zweiten Reaktionsstufe in einem Temperaturbereich von 20 bis 40°C, bevorzugt 25 bis 35°C durchgeführt.

Als inertes Lösungsmittel kommt ein aliphatischer, aromatischer oder alkylaromatischer Kohlenwasserstoff, ein halogenierter aromatischer Kohlenwasserstoff oder zusätzlicher Benzylalkohol über die genannten 2 bis 5 Mol hinaus in Frage. Beispiele für solche Lösungsmittel sind: Pentan, Hexan, Heptan, Octan, Decan, Dodecan und höhere geradkettige oder verzweigte aliphatische Kohlenwasserstoffe, sowie Gemische daraus, wie Ligroin oder Petrolether, Benzol, Toluol, Ethylbenzol, Chlorbenzol, Brombenzol, Dichlorbenzol, Dibrombenzol, Chlortoluol, Dichlortoluol oder Cyclohexan. Selbstverständlich können auch Gemische mehrerer der genannten Lösungsmittel eingesetzt werden.

In einer bevorzugten Ausführungsform kommt Benzylalkohol gleichzeitig als Reaktionspartner und Lösungsmittel zum Einsatz. Hierbei wird Benzylalkohol in einer Menge von 3 bis 20 Gew.-Teilen, bevorzugt 5 bis 15 Gew -Teilen, bezogen auf 1 Gew.-Teil Dichlor-dinitrobenzol, eingesetzt Selbstverständlich kann der Benzylalkohol auch im Gemisch mit den genannten inerten Lösungsmitteln verwendet werden.

Die Reaktion ist unabhängig vom Reaktionsdruck und wird daher zweckmäßig bei Normaldruck durchgeführt. Ein erhöhter Druck könnte dann sinnvoll sein, wenn ein niedrig siedendes Lösungsmittel verwendet wird. In diesem Falle wird bevorzugt unter dem sich automatisch einstellenden Eigendruck gearbeitet. Ein verminderter Druck ist dann zweckmäßig, wenn die Temperatur-gestufte Reaktion in der ersten und/oder zweiten Stufe bei einer konstanten Siedetemperatur unter Rückfluß durchgeführt werden soll.

Die Temperatur-gestufte Reaktion von Dichlor-dinitrobenzol mit Benzylalkohol in Gegenwart der genannten Base erfordert in der ersten Stufe bei einer Reaktionstemperatur von 5 bis 15°C im allgemeinen Reaktionszeiten von 15 Minuten bis 3 Stunden, in der zweiten Stufe bei einer Reaktionstemperatur von 25 bis 35°C im allgemeinen Reaktionszeiten von 30 Minuten bis 6 Stunden und ergibt hohe Ausbeuten, die 90 % überschreiten.

Das Reaktionsprodukt ist im Reaktionsmedium schwerlöslich und kann daher durch einfaches Filtrieren isoliert werden. Anschließend können die anorganischen Nebenprodukte (beispielsweise Alkali- und/oder Erdalkalichloride) durch Anschlämmen in Wasser und erneute Filtration entfernt werden.

In das erfindungsgemäße Verfahren kann jedoch auch anderes reines 1,3-Dibenzyloxy-4,6-dinitrobenzol eingesetzt werden, das auf jede beliebige Weise hergestellt wurde.

Wesentlich für die Durchführung des erfindungsgemäßen Verfahrens ist, daß man das 1,3-Dibenzyloxy-4,6-dinitrobenzol in einem zweiphasigen Gemisch aus verdünnter wäßriger Salzsäure und einem mit verdünnter wäßriger Salzsäure nicht oder nur begrenzt mischbaren organischen Lösungsmittel bei normalem oder erhöhtem Druck und normaler oder erhöhter Temperatur durch eine katalytische Hydrierung, bevorzugt katalytische Pumphydrierung, zum 4,6-Diamino-resorcin-dihydrochlorid umsetzt. Bei der bevorzugten Ausführungsform wird eine Lösung oder Suspension von 1,3-Dibenzyloxy-4,6-dinitrobenzol in einem organischen Lösungsmittel unter Hydrierbedingungen in einen Hydrierautoklaven eingepumpt, in dem eine Suspension des Edelmetall-Kontaktes in verdünnter wäßriger Salzsäure oder einer wäßrigen, salzsauren Lösung von 4,6-Diamino-resorcin-dihydrochlorid unter H₂-Druck vorgelegt ist.

Bei der katalytischen Hydrierung von 1,3-Dibenzyloxy-4,6-dinitrobenzol in dem zweiphasigen Gemisch aus verdünnter wäßriger Salzsäure und einem mit verdünnter wäßriger Salzsäure nicht oder nur begrenzt mischbaren organischen Lösungsmittel zu 4,6-Diamino-resorcin erfolgt eine Reduktion der Nitrogruppen zu den Aminogruppen und eine Abspaltung der Benzylgruppen unter Bildung von Toluol sowie eine Stabilisierung des extrem oxidationsempfindlichen Diaminoresorcins in Form des Dihydrochlorids. Als Kontakte/Katalysatoren werden Platinmetalle, wie Palladium, Platin, Rhodium oder Ruthenium, bevorzugt Palladium oder Platin in einer Menge von 0,1 bis 10 Gew.-%, bezogen auf die Gesamtmenge Kontakt, auf einem geeigneten, gegenüber wäßriger Salzsäure stabilen Träger eingesetzt. Geeignete Träger sind A-Kohle, SiO₂, und andere dem Fachmann bekannte. Ganz besonders bevorzugt wird ein Palladium/A-Kohle-Kontakt eingesetzt. Der Platinmetall-Träger-Kontakt wird in einer Menge von 0,1 bis 10 Gew.-%, gerechnet als Platinmetall, bevorzugt 0,2 bis 2 Gew.-%, bezogen auf das Gewicht von 1,3-Dibenzyloxy-4,6-dinitrobenzol, eingesetzt. Die Hydrierung wird bei einem H₂-Druck von 1 bis 200 bar, bevorzugt 5 bis 50 bar, insbesondere 10 bis 30 bar und im Temperaturbereich von 0 bis 200°C, bevorzugt 20 bis 150°C, insbesondere 40 bis 120°C durchgeführt.

Bei der Hydrierung, die sich auch drucklos durchführen läßt, kann der Wasserstoff durch geeignete, H₂-abgebende Substanzen, beispielsweise Hydrazin, Hydrazinhydrat, Hydrazinium-Salze, Ameisensäure, Formiate oder mehrere von ihnen ersetzt werden.

Die Konzentration und Menge der verdünnten wäßrigen Salzsäure, die man in dem Hydrier-Autoklaven vorlegt, wird im erfindungsgemäßen Verfahren zweckmäßigerweise so gewählt, daß das gebildete 4,6-Diamino-resorcin-dihydrochlorid bei der gewählten Reaktionstemperatur in der wäßrigen Phase vollständig gelöst ist, und beträgt im allgemeinen 0,1 bis 20 Gew.-%, bevorzugt 1 bis 15 Gew.-%, besonders bevorzugt 3 bis 12 Gew.-% und 1 bis 20 g pro g eingesetztem 1,3-Dibenzyloxy-4,6-dinitrobenzol, bevorzugt 3 bis 10 g/g.

Als mit verdünnter wäßriger Salzsäure nicht mischbares organisches Lösungsmittel kann für die Hydrierung ein geeigneter Alkohol, ein Ether, ein aromatischer oder alkylaromatischer Kohlenwasserstoff, eine geeignete organische Säure oder ein Gemisch mehrerer von ihnen eingesetzt werden Beispiele für solche flüssigen Reaktionsmedien sind Propanol, Isopropylalkohol, Butanol, Isobutanol, Methyl-tert.-butylether, Benzol, Toluol, Xylol, Propionsäure oder ein Gemisch mehrerer von ihnen. Wesentlich ist jedoch, daß das mit verdünnter wäßriger Salzsäure nicht oder nur begrenzt mischbare organische Lösungsmittel eine gute Löslichkeit für Wasserstoff und eine gute Benetzbarkeit des vorzugsweise verwendeten Palladium/A-Kohle-Kontaktes besitzt. Für die bevorzugte katalytische Pumphydrierung ist es außerdem wesentlich, daß das mit verdünnter wäßriger Salzsäure nicht oder nur begrenzt mischbare organische Lösungsmittel eine ausreichende Löslichkeit für das 1,3-Dibenzyloxy-4,6-dinitrobenzol besitzt, um in einer besonders wirtschaftlichen Weise Lösungen von 1,3-Dibenzyloxy-4,6-dinitrobenzol in den Hydrierautoklaven portionsweise oder gleichmäßig einpumpen zu können. Bevorzugt werden aromatische oder alkylaromatische Kohlenwasserstoffe wie Benzol, Toluol oder Xylol als mit verdünnter wäßriger Salzsäure nicht mischbares organisches Lösungsmittel eingesetzt. Ganz besonders bevorzugt wird Toluol eingesetzt, das durch Abspaltung der Benzylgruppen aus dem 1,3-Dibenzyloxy-4,6-dinitrobenzol ohnehin gebildet wird, was zu einer besonders wirtschaftlichen Aufarbeitung des zweiphasigen Reaktionsgemisches aus der Hydrierung durch einfache Phasentrennung und Wiederverwendung des Toluols ohne destillative Reinigung führt. Das gesamte mit verdünnter wäßriger Salzsäure nicht oder nur begrenzt mischbare organische Lösungsmittel für die Hydrierung wird in einer Menge von 0,5 bis 50 Gew.-Teilen, bevorzugt 1 bis 10 Gew.-Teilen, bezogen auf 1 Gew.-Teil l,3-Dibenzyloxy-4,6-dinitrobenzol eingesetzt. Selbstverständlich kann das organische Lösungsmittel auch schon teilweise gemeinsam mit der verdünnten, wäßrigen Salzsäure im Hydrierautoklaven vorgelegt werden.

Im einzelnen wird das erfindungsgemäße Verfahren beispielsweise wie folgt durchgeführt. In einem Hydrier-Autoklaven aus einem gegenüber wäßriger Salzsäure beständigen Werkstoff wird eine Suspension von Pd (5 %)/A-Kohle-Kontakt in 1 bis 30 gew.-%iger Salzsäure vorgelegt Unter bevorzugten Hydrierbedingungen (60 bis 80°C; 10 bis 20 bar H₂) wird im Verlaufe von 30 bis 90 Minuten eine 10 bis 80 gew.-%ige Lösung bzw. Suspension von 1,3-Dibenzyloxy-4,6-dinitrobenzol in Toluol eingepumpt, wobei die Konzentration von 1,3-Dibenzyloxy-4,6-dinitrobenzol bezogen auf die Gesamtmenge Toluol und wäßriger Salzsäure, 5 bis 20 Gew.-% und das Gewichtsverhältnis 1,3-Dibenzyloxy-4,6-dinitrobenzol zu Palladium beispielsweise 85:1 beträgt Nach beendeter Wasserstoff-Aufnahme wird die Reaktionsmischung beispielsweise 60 Minuten bei 60 bis 80°C und 10 bis 20 bar H₂ nachgerührt, der Rührer stillgesetzt und ca. 66 % der zweiphasigen Reaktionsmischung aus Toluol und wäßriger, salzsaurer 4,6-Diamino-resorcin-dihydrochlorid-Lösung aus dem Hydrier-Autoklaven mittels Stickstoff über ein aus korrosionsbeständigem Material bestehendes Steigrohr, das mit einer Fritte versehen ist, in eine Vorlage abgedrückt. Nach Abtrennen des organischen Lösungsmittels (Toluol) durch einfache Phasentrennung kann das stabile 4,6-Diaminoresorcin-dihydrochlorid aus der resultierenden salzsauren, wäßrigen Lösung, gegebenenfalls nach Toluol-Stripping und A-Kohle-Klärung, in der üblichen Weise beispielsweise durch Einengen der wäßrigen Lösung und/oder durch Ausfällen mit konzentrierter Salzsäure und/oder durch Einleiten von gasförmigem HCl und/oder gegebenenfalls durch Aussalzen, beispielsweise mit NaCl, oder auf jede beliebige, dem Fachmann bekannte Weise gegebenenfalls nach vorheriger Zugabe eines Stabilisators, beispielsweise SnCl₂, isoliert werden. Auf die gleiche Weise können mit dem eingesetzten Pd (5 %)/A-Kohle-Kontakt mehrere Hydrierungen nacheinander ohne Abnahme der Katalysator-Aktivität durchgeführt werden, was zu einem sehr niedrigen Katalysator-Verbrauch führt.

Es ist als überraschend anzusehen, daß nach dem erfindungsgemäßen Verfahren insbesondere bei der bevorzugten katalytischen Pumphydrierung mehrere Hydrierungen nacheinander ohne Abnahme der Katalysator-Aktivität durchgeführt werden können, und daß das Diamino-resorcin-dihydrochlorid in sehr hoher Ausbeute und ausgezeichneter Qualität ohne weitere Reinigung als farbloses Produkt in einer besonders wirtschaftlichen Weise erhalten werden kann.

Das erfindungsgemäße Verfahren kann diskontinuierlich in der beschriebenen Weise, beispielsweise als Pumphydrierung, oder kontinuierlich, beispielsweise in Schlaufenreaktoren durchgeführt werden.

### Beispiel 1

In einem emaillierten 3 1-Hydrier-Autoklaven wurde eine Suspension von 25 g feuchtem Pd(5 %)/A-Kohle-Kontakt (20 g trocken; 1 g Pd) in 770 g 5,5 gew.-%iger wäßriger Salzsäure vorgelegt. Unter Hydrierbedingungen wurden bei 80°C und 10 bis 20 bar H₂ im Verlaufe von ca. 30 min. 1140 g 10 gew.-%ige 1,3-Dibenzyloxy-4,6-dinitrobenzol/Toluol-Suspension (0,3 mol) eingepumpt. Die Reaktionsmischung wurde ca. 1 h bis zur beendeten Wasserstoff-Aufnahme bei 80°C und 10 bis 20 bar H₂ nachgerührt, der Rührer stillgesetzt und ca. 1275 g (66 %) der zweiphasigen Reaktionsmischung aus Toluol und wäßriger, salzsaurer 4,6-Diamino-resorcin-dihydrochlorid-Lösung aus dem Hydrier-Autoklaven mittels Stickstoff über ein Steigrohr, das mit einer Sintermetall-Fritte versehen war, in eine Vorlage abgedrückt. Nach Abtrennen von ca. 720 g farblosem Toluol durch einfache Phasentrennung (obere Phase) wurden ca. 555 g einer gelben, salzsauren, wäßrigen Lösung von 4,6-Diamino-resorcin-dihydrochlorid erhalten. Nach Abtrennung geringer Mengen Toluol durch Anstrippen der wäßrigen Lösung und gegebenenfalls A-Kohle-Klärung gegebenenfalls nach Zugabe geringer Mengen SnCl₂ · 2 H₂O wurde durch Zugabe von ca. 555 g 37 gew.-%iger Salzsäure bei 20 bis 60°C das 4,6-Diamino-resorcin-dihydrochlorid ausgefällt, die resultierende Suspension auf Raumtemperatur (20°C) kaltgerührt, das sehr gut filtrierende Produkt über eine Glassinternutsche isoliert und im Vakuum (100 mbar) bei 20 bis 60°C getrocknet.

In die im Hydrier-Autoklaven verbliebene Suspension des Pd (5 %)/A-Kohle-Kontaktes in ca. 635 g zweiphasiger Mischung aus Toluol und wäßriger, salzsaurer 4,6-Diamino-resorcin-dihydrochlorid-Lösung wurden auf die gleiche Weise zunächst weitere 480 g 6 gew.-%ige wäßrige Salzsäure und anschließend 760 g 10 gew.-%ige 1,3-Dibenzyloxy-4,6-dinitrobenzol/Toluol-Suspension (0,3 mol) bei 80°C und 10 bis 20 bar H₂ im Verlaufe von ca. 30 min. eingepumpt und ohne Abnahme der Katalysator-Aktivität hydriert. Auf diese Weise wurden insgesamt 10 Hydrierungen mit 9 Kontakt-Rückführungen nacheinander durchgeführt.

Es wurden insgesamt erhalten:
427 g 4,6-Diamino-resorcin-dihydrochlorid, trocken.

Nach HPLC (High Performance Liquid Chromatography) war das eingesetzte 1,3-Dibenzyloxy-4,6-dinitrobenzol vollständig umgesetzt und das isolierte 4,6-Diamino-resorcin-dihydrochlorid chromatographisch praktisch einheitlich. Nach ¹HNMR enthielten die isolierten Produkte keine Benzyl-Gruppen.

| | |
|---|---|
| Gehalt (aus Chlor) MG 213 | 99,7 Gew.-% |
| Gehalt (aus Stickstoff) MG 213 | 99,7 Gew.-% |
| Gehalt (aus Kohlenstoff) MG 213 | 99,5 Gew.-% |
| Gehalt (aus HPLC) MG 213 | 99,1 Gew.-% |

Die Ausbeute an 4,6-Diamino-resorcin betrug 95 % der theoretischen Ausbeute (aus Kohlenstoff), bezogen auf eingesetztes 1,3-Dibenzyloxy-4,6-dinitrobenzol.

Das in das erfindungsgemäße Verfahren eingesetzte reine 1,3-Dibenzyloxy-4,6-dinitrobenzol wurde vorzugsweise auf folgendem Wege hergestellt:

### Stufe a): techn. 1,3-Dichlor-4,6/2,4-dinitrobenzol-Gemisch:

In einer Mehrhalskolben-Rührapparatur mit Innenthermometer und zwei Dosiertropftrichtern für 1,3-Dichlorbenzol und Mischsäure wurden 700 g Schwefelsäure-Monohydrat (7,14 mol) vorgelegt. Unter Rühren und Eis-Kühlung wurden simultan im Verlaufe von 1 h bei 20°C 147 g 1,3-Dichlorbenzol, 99,4 %ig (0,99 mol), und 506 g Mischsäure (2,20 mol HNO₃) eindosiert, wobei nach ca. 30 Minuten die Kristallisation des Nitrierproduktes einsetzte. Die Reaktionsmischung wurde 4 h bei 20°C nachgerührt. In eine Vorlage aus 3640 g Eis/Wasser-Gemisch wurde die Reaktionsmischung unter Rühren bis zu einer maximalen Temperatur von 20°C im Verlaufe von ca. 30 Minuten eingetragen. Die resultierende Produkt-Suspension wurde 1 h bei 20°C nachgerührt, das sehr gut filtrierende Reaktionsprodukt über eine Glassinternutsche isoliert und mit insgesamt ca. 3750 g Wasser säurefrei gewaschen und bei 40°C im Vakuum getrocknet.

Es wurden erhalten:
226 g 1,3-Dichlor-4,6-dinitrobenzol, trocken.

Der durch GC bestimmte Gehalt des isolierten Produktes betrug:

| | |
|---|---|
| 89,7 Gew.-% | 1,3-Dichlor-4,6-dinitrobenzol |
| 10,1 Gew.-% | 1,3-Dichlor-2,4-dinitrobenzol |
| 0,1 Gew.-% | 1,3-Dichlor-2-nitrobenzol |
| 0,1 Gew.-% | 1,3-Dichlor-4-nitrobenzol |

Die Ausbeute an 1,3-Dichlor-4,6-dinitrobenzol in Form des 4,6/2,4-Dinitro-Gemisches betrug 86,4 % der theoretischen Ausbeute, bezogen auf eingesetztes 1,3-Dichlorbenzol.

Die Mischsäure wurde wie folgt hergestellt:
141,4 g = 2,20 mol HNO₃, 98 %ig, wurden vorgelegt. Unter Eis-Kühlung und Rühren wurden bis zu einer maximalen Temperatur von 35°C 365 g Oleum 65 (3,0 mol SO₃; 1,3 mol H₂SO₄) im Verlaufe von mehreren Stunden eingetropft.

### Stufe b): 1,3-Dibenzyloxy-4,6-dinitrobenzol:

In einer Mehrhalskolben-Rührapparatur mit Innenthermometer und Feststoff-Eintrag wurden 1560 g Benzylalkohol vorgelegt. Unter Durchleiten von trockenem Stickstoff und Eis-Kühlung wurden im Verlaufe von ca. 15 Minuten bis zu einer maximalen Temperatur von 25°C 74 g gemahlenes NaOH (1,85 mol) eingetragen. Die Reaktionsmischung wurde bis zur vollständigen Lösung ca. 12 h bei Raumtemperatur (20°C) nachgerührt. In die gelblich gefärbte, schwach getrübte Reaktionslösung wurden unter Durchleiten von trockenem Stickstoff und Eis/NaCl-Kühlung im Verlaufe von ca. 30 Minuten bei 10°C 183 g 1,3-Dichlor-4,6/2,4-dinitrobenzol-Isomerengemisch (0,685 mol 1,3-Dichlor-4,6-dinitrobenzol, 0,085 mol 1,3-Dichlor-2,4-dinitrobenzol) eingetragen. Die resultierende hellgelbe Suspension wurde 30 Minuten bei 10°C nachgerührt, auf 30°C erwärmt und ca. 3 h bei dieser Temperatur nachgerührt. Das gut filtrierende Produkt-Gemisch aus 1,3-Dibenzyloxy-4,6-dinitrobenzol und NaCl wurde bei 30°C über eine Glassinternutsche isoliert und zur Entfernung der Benzylalkohol-Mutterlauge gut trocken gesaugt. In eine Vorlage aus 2500 g Wasser wurde unter Rühren das isolierte Rohprodukt bei 20°C eingetragen, die resultierende Anschlämmung bis zur vollständigen Lösung von NaCl ca. 1 h bei 20°C gerührt, das gereinigte, praktisch farblose Produkt erneut filtriert, zur vollständigen Entfernung von Benzylalkohol mit ca. 5000 g Wasser in mehreren Portionen gewaschen und gegebenenfalls unter vermindertem Druck bei 60°C getrocknet.

Es wurden erhalten:
237 g Reinprodukt, trocken

Der durch HPLC bestimmte Gehalt des isolierten Produktes betrug:

| | |
|---|---|
| Gehalt (Titan-Red.) MG 380 HPLC | 99,8 Gew.-% |
| 1,3-Dibenzyloxy-4,6-dinitrobenzol | 99,1 Gew.-% |
| 1-Benzyloxy-3-chlor-4,6-dinitrobenzol | 0,6 Gew.-% |
| 1,3-Dibenzyloxy-2,4-dinitrobenzol | <0,1 Gew.-% |

Die Ausbeute an 1,3-Dibenzyloxy-4,6-dinitrobenzol betrug 91,0 % der theoretischen Ausbeute, bezogen auf eingesetztes 1,3-Dichlor-4,6-dinitrobenzol, in Form des 1,3-Dichlor-4,6/2,4-dinitrobenzol-Gemisches.

Der Benzaldehyd-Gehalt in der Benzylalkohol-Mutterlauge betrug nach GC 0,5 %.

## Patentansprüche

1. Verfahren zur Herstellung von 4,6-Diamino-resorcin-dihydrochlorid aus 1,3-Dibenzyloxy-4,6-dinitrobenzol, dadurch gekennzeichnet, daß man 1,3-Dibenzyloxy-4,6-dinitrobenzol an einem Edelmetall-Kontakt mit Wasserstoff in einem zweiphasigen Gemisch aus verdünnter wäßriger Salzsäure und einem mit verdünnter wäßriger Salzsäure nicht oder nur begrenzt mischbaren organischen Lösungsmittel bei 1 bis 200 bar und 0 bis 200°C katalytisch hydriert.

2. Verfahren nach Anspruch 1, dadurch gekennzeichnet, daß das mit verdünnter wäßriger Salzsäsure nicht mischbare organische Lösungsmittel in einer Menge von 0,5 bis 50 Gew.-Teilen, bevorzugt 1 bis 10 Gew.-Teilen, bezogen auf 1 Gew.-Teil 1,3-Dibenzyloxy-4,6-dinitrobenzol eingesetzt wird.

3. Verfahren nach Anspruch 1, dadurch gekennzeichnet, daß als mit verdünnter wäßriger Salzsäure nicht mischbare organische Lösungsmittel aromatische oder alkylaromatische Kohlenwasserstoffe, bevorzugt Toluol eingesetzt werden.

4. Verfahren nach Anspruch 1, dadurch gekennzeichnet, daß als Kontakt ein Platinmetall, bevorzugt Palladium, auf einem gegenüber wäßriger Salzsäure stabilen Träger eingesetzt wird.

5. Verfahren nach Anspruch 1, dadurch gekennzeichnet, daß der Kontakt in einer Menge von 0,1 bis 10 Gew.-%, gerechnet als Platinmetall, bevorzugt 0,2 bis 2 Gew.-%, bezogen auf das Gewicht von 1,3-Dibenzyloxy-4,6-dinitrobenzol, eingesetzt wird.

6. Verfahren nach Anspruch 4, dadurch gekennzeichnet, daß als Kontakt Palladium auf A-Kohle eingesetzt wird.

7. Verfahren nach Anspruch 1, dadurch gekennzeichnet, daß die Hydrierung bei einem H₂-Druck von 5 bis 50 bar, insbesondere 10 bis 30 bar und im Temperaturbereich von 20 bis 150°C, insbesondere 40 bis 120°C durchgeführt wird.

8. Verfahren nach Anspruch 1, dadurch gekennzeichnet, daß bei druckloser Hydrierung der Wasserstoff durch H₂-abgebende Substanzen, bevorzugt durch Hydrazin, Hydrazin-hydrat, Hydrazinium-Salze, Ameisensäure, Formiate oder mehrere von ihnen ersetzt wird.

9. Verfahren nach Anspruch 1, dadurch gekennzeichnet, daß die Hydrierung in der Form der Pumphydrierung durchgeführt wird.

## Claims

1. Process for preparing 4,6-diamino-resorcinol dihydrochloride from 1,3-dibenzyloxy-4,6-dinitrobenzene, characterized in that 1,3-dibenzyloxy-4,6-dinitrobenzene is catalytically hydrogenated over a noble metal catalyst with hydrogen in a two-phase mixture of dilute aqueous hydrochloric acid and an organic solvent which is immiscible or miscible only to a limited extent with dilute aqueous hydrochloric acid, at 1 to 200 bar and 0 to 200°C.

2. Process according to Claim 1, characterized in that the organic solvent immiscible with dilute aqueous hydrochloric acid is employed in a quantity of 0.5 to 50 parts by weight, preferably 1 to 10 parts by weight, relative to 1 part by weight of 1,3-dibenzyloxy-4,6-dinitrobenzene.

3. Process according to Claim 1, characterized in that aromatic or alkylaromatic hydrocarbons, preferably toluene, are employed as the organic solvent immiscible with dilute aqueous hydrochloric acid.

4. Process according to Claim 1, characterized in that the catalyst employed is a platinum metal, preferably palladium, on a support which is stable towards aqueous hydrochloric acid.

5. Process according to Claim 1, characterized in that the catalyst is employed in a quantity of 0.1 to 10% by weight, calculated as platinum metal, preferably 0.2 to 2% by weight, relative to the weight of 1,3-dibenzyloxy-4,6-dinitrobenzene.

6. Process according to Claim 4, characterized in that the catalyst employed is palladium on activated carbon.

7. Process according to Claim 1, characterized in that the hydrogenation is carried out at an H₂ pressure of 5 to 50 bar, in particular 10 to 30 bar, and in a temperature range from 20 to 150°C, in particular 40 to 120°C.

8. Process according to Claim 1, characterized in that, in the case of unpressurized hydrogenation, the hydrogen is replaced by H₂-releasing substances, preferably by hydrazine, hydrazine hydrate, hydrazinium salts, formic acid, formates or two or more of these.

9. Process according to Claim 1, characterized in that the hydrogenation is carried out in the form of pump hydrogenation.

## Revendications

1. Procédé de préparation de dichlorhydrate de 4,6-diaminorésorcine à partir de 1,3-dibenzyloxy-4,6-dinitrobenzène caractérisé en ce que l'on hydrogène catalytiquement à 1 à 200 bar et 0 à 200°C du 1,3-dibenzyloxy-4,6-dinitrobenzène sur un catalyseur de métal noble avec de l'hydrogène dans un mélange à deux phases d'acide chlorhydrique aqueux dilué et d'un solvant organique immiscible ou miscible de manière seulement limitée à l'acide chlorhydrique aqueux dilué.

2. Procédé selon la revendication 1, caractérisé en ce que le solvant organique immiscible à l'acide chlorhydrique aqueux dilué est utilisé en une quantité de 0,5 à 50 parties en masse, de préférence de 1 à 10 parties en masse, par partie en masse de 1,3-dibenzyloxy-4,6-dinitrobenzène.

3. Procédé selon la revendication 1, caractérisé en ce que l'on utilise comme solvant organique immiscible à l'acide chlorhydrique aqueux dilué des hydrocarbures aromatiques ou alkylaromatiques, de préférence le toluène.

4. Procédé selon la revendication 1, caractérisé en ce que l'on utilise comme catalyseur un platinoïde, de préférence le palladium, sur un support stable à l'acide chlorhydrique aqueux dilué.

5. Procédé selon la revendication 1, caractérisé en ce que le catalyseur est utilisé en une quantité de 0,1 à 10 % en masse, calculée en platinoïde, de préférence de 0,2 à 2 % en masse, par rapport à la masse de 1,3-dibenzyloxy-4,6-dinitrobenzène.

6. Procédé selon la revendication 4, caractérisé en ce que l'on utilise comme catalyseur le palladium sur charbon A.

7. Procédé selon la revendication 1, caractérisé en ce que l'hydrogénation est réalisée à une pression de H₂ de 5 à 50 bar, en particulier de 10 à 30 bar, et dans le domaine de température de 20 à 150°C, en particulier de 40 à 120°C.

8. Procédé selon la revendication 1, caractérisé en ce que, dans le cas de l'hydrogénation sans pression, l'hydrogène est remplacé par des substances libérant H₂, de préférence par l'hydrazine, l'hydrate d'hydrazine, des sels d'hydrazinium, l'acide formique, des formiates ou plusieurs de celles-ci.

9. Procédé selon la revendication 1, caractérisé en ce que l'hydrogénation est réalisée sous forme d'hydrogénation par pompage.
